# EUROPEAN PATENT APPLICATION

(11) **EP 4 671 243 A1**
(43) Date of publication of application: **31.12.2025**
(21) Application number: 24921233.3
(22) Date of filing: 24.05.2024
(51) Int. Cl.: C07D 471/04, C07C 255/57

(54) **FINERENONE, PREPARATION METHOD THEREFOR AND FINERENONE INTERMEDIATE**

(30) Priority: 30.01.2024 CN 202410121511
(71) Applicant: Zhejiang Shenzhou Pharmaceutical Co., Ltd., Taizhou, Zhejiang 317306 (CN)
(72) Inventor: WANG, Rong, Taizhou, Zhejiang 317306 (CN); SHAO, Zhenping, Taizhou, Zhejiang 317306 (CN); WANG, Bingqian, Taizhou, Zhejiang 317306 (CN); WANG, Zhaoyang, Taizhou, Zhejiang 317306 (CN); WANG, Youfu, Taizhou, Zhejiang 317306 (CN)
(74) Representative: Kirkpatrick
(86) International application number: PCT/CN2024/095247
(87) International publication number: WO 2025/161191

(57) **Abstract**

The present application belongs to the technical field of preparation and processing of drugs, and particularly relates to finerenone, a preparation method therefor and a finerenone intermediate. In the preparation method provided by the present application, a compound with a diester structure as represented by formula 2 is used as a reaction raw material to carry out a series of reactions, a chiral catalyst is used in the reaction to obtain a product with an S/R configuration ratio of up to 85:15, and the product is resolved by means of using a tartaric acid derivative, so as to prepare a compound with a structure as represented by formula 7, and then the resulting compound is subjected to hydrolysis and amidation reactions to obtain finerenone. A finerenone drug substance with an ee value of >99.9% and a purity of up to 99.9% can be obtained by means of the preparation method of the present application. Moreover, the reactions during the preparation method provided by the present application are all common reactions, not involving dangerous reaction steps such as hydrogenolysis and high-pressure processes. The method has mild reaction conditions, generates no highly toxic by-products, has low requirements for reaction equipment, has low operational costs, and is simple and convenient to operate. Therefore, the method is suitable for industrial production, and has good market prospects.

## Description

### TECHNICAL FIELD

The present disclosure belongs to the technical field of pharmaceutical preparation and processing, and in particular to finerenone and a preparation method thereof, and a finerenone intermediate.

### BACKGROUND

Finerenone, with the chemical name (4S)-4-(4-cyano-2-methoxyphenyl)-5-ethoxy-1,4-dihydro-2,8-dimethyl-1,6-naphthyridine-3-carboxamide and the molecular formula C₂₁H₂₂N₄O₃, is a non-steroidal, selective mineralocorticoid receptor antagonist. Studies have shown that finerenone blocks the harmful effects caused by overactivation of the mineralocorticoid receptor and is used for the treatment of adult patients with chronic kidney disease accompanied by type 2 diabetes.

Numerous literature reports have detailed preparation methods for finerenone, with the most frequently reported synthetic route being as follows:

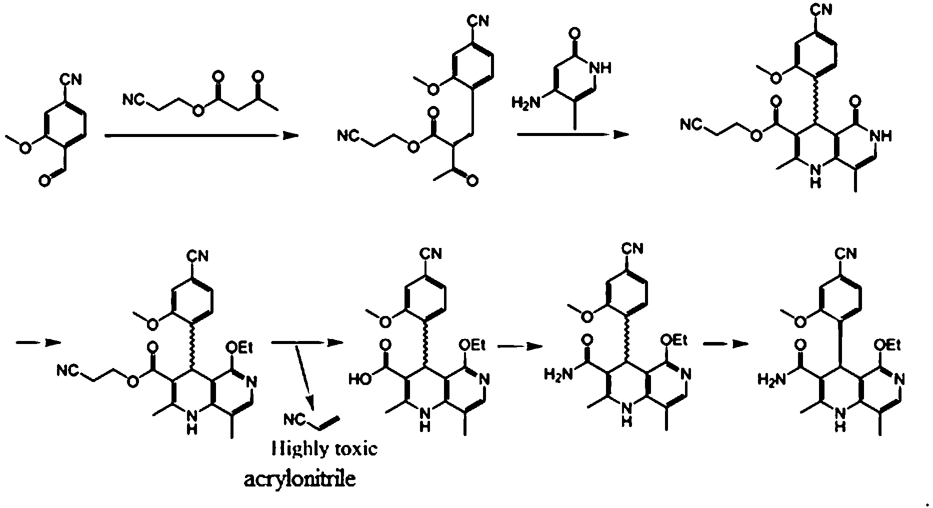

However, since the above preparation method uses 3-oxobutanoic acid 2-cyanoethyl ester, highly toxic acrylonitrile is generated during the hydrolysis. Furthermore, this route requires high-pressure reactions, presenting a high-risk factor and making it unsuitable for scale-up production.

Another method for preparing the finerenone active pharmaceutical ingredient (API) reported in the literature follows the synthetic route below:

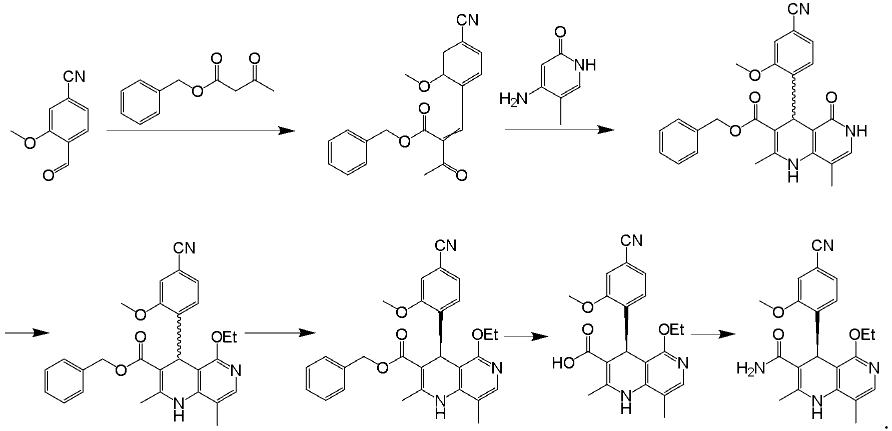

This route employs a benzyl ester to protect the carboxylic acid. Conventional methods struggle to hydrolyze the benzyl ester, necessitating the use of hydrogen to allow hydrogenolysis to obtain the required carboxylic acid intermediate. The hydrogen constitutes a hazardous reaction, unsuitable for scale-up production. Moreover, during the hydrogenolysis, expensive palladium catalysts are required, while side reactions occur at the cyano group on the benzene ring, introducing undesirable impurities.

In summary, the preparation methods of finerenone disclosed in the above two synthetic routes suffer from issues such as highly toxic byproducts, expensive palladium catalysts, and dangerous reaction conditions. Additionally, the S/R configuration ratio obtained in both cases is 1:1. After resolving to obtain the desired configuration product, the other half of the isomer cannot be effectively utilized, resulting in waste and increased costs. Therefore, there is an urgent need to establish a method for preparing finerenone that offers high yield, simple operation, and mild conditions.

### SUMMARY

An object of the present disclosure is to provide finerenone and a preparation method thereof, and a finerenone intermediate. The preparation method features mild reaction conditions, absence of highly toxic byproducts, low requirements for reaction equipment and operational costs, and simple operation. Furthermore, the preparation method achieves a higher proportion of the desired configuration relative to the undesired configuration, effectively improving the yield of the desired S-configuration product obtained through resolution. Therefore, the preparation method is suitable for industrial production and demonstrates enhanced market prospects.

To achieve the above object, the present disclosure provides the following technical solutions:
The present disclosure provides a method for preparing finerenone, including the following steps:
I) mixing a compound of Formula 1, a compound of Formula 2, an organic base, acetic acid, and a first organic solvent, and conducting condensation, to obtain a compound of Formula 3;
II) mixing the compound of Formula 3, a compound of Formula 4, a chiral catalyst, and a second organic solvent, and conducting cyclization, to obtain a compound of Formula 5;
III) mixing the compound of Formula 5, triethyl orthoformate, concentrated sulfuric acid, and a third organic solvent, and conducting etherification, to obtain a compound of Formula 6;
IV) mixing the compound of Formula 6, a tartaric acid derivative, and a fourth organic solvent, conducting a first heat preservation by maintaining an obtained mixture at a first temperature, and then cooling a resulting heated product to a second temperature and conducting second heat preservation, to obtain a salt of the tartaric acid derivative; and mixing the salt of the tartaric acid derivative, water, and a fifth organic solvent, and then regulating a pH value of a resulting mixture to 7.5 to 9.5 by using a basic pH regulator, to obtain a compound of Formula 7;
V) mixing the compound of Formula 7, a sixth organic solvent, and a basic compound, and conducting hydrolysis, to obtain a compound of Formula 8; and
VI) mixing the compound of Formula 8, N,N-carbonyldiimidazole (CDI), a seventh organic solvent, 4-dimethylaminopyridine (DMAP), and aqueous ammonia, and conducting amidation, to obtain the finerenone; 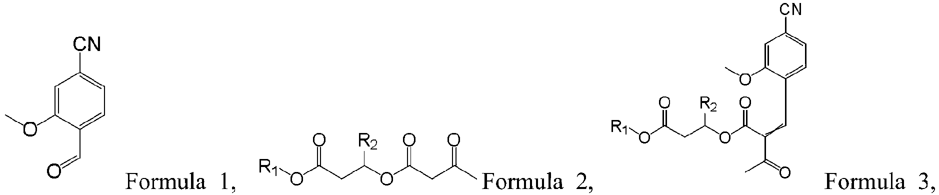 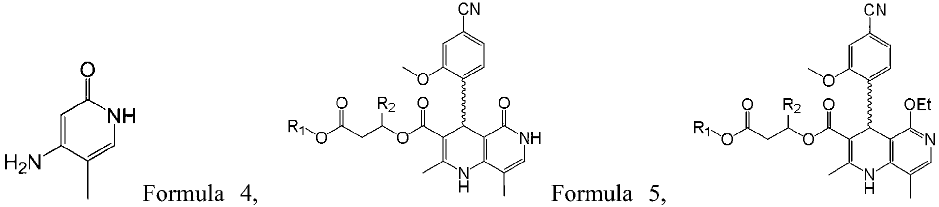 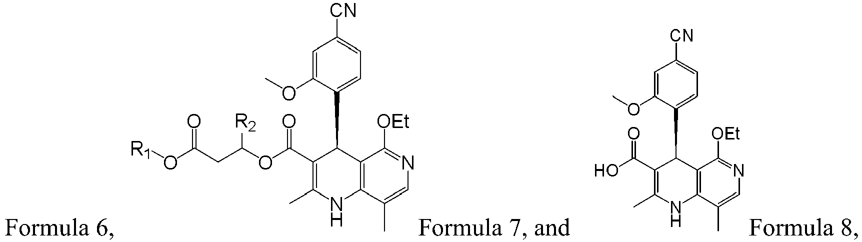 where in Formula 2, Formula 3, Formula 5, Formula 6, and Formula 7, R₁ is C1-10 alkyl, R₂ is selected from the group consisting of hydrogen, C1-10 alkyl, halogen-substituted C1-10 alkyl, and cyano-substituted C1-10 alkyl.

In some embodiments, in step I),
the organic base is at least one selected from the group consisting of piperidine, morpholine, and pyrrolidine;
the first organic solvent is at least one selected from the group consisting of methanol, ethanol, n-propanol, isopropanol, n-butanol, and sec-butanol;
a mass ratio of the compound of Formula 1, the organic base, the acetic acid, and the compound of Formula 2 is in a range of 1:0.03-0.2:0.02-0.2:1.0-2.0; and
the condensation is conducted at a temperature of 20°C to 50°C.

In some embodiments, in step II),
the chiral catalyst is selected from the group consisting of D-(+)-10-camphorsulfonic acid and (S)-(+)-1,1'-binaphthyl-2,2'-diyl hydrogenphosphate;
the second organic solvent is at least one selected from the group consisting of isopropanol, tert-butanol, sec-butanol, and sec-amyl alcohol;
a mass ratio of the compound of Formula 3, the compound of Formula 4, and the chiral catalyst is in a range of 1:0.2-1.2:0.2-2.0; and
the cyclization is conducted at a temperature of 70°C to 120°C.

In some embodiments, in step III),
the third organic solvent is at least one selected from the group consisting of *N,N-*dimethylformamide (DMF), *N*,*N*-dimethylacetamide (DMA), and *N*-methylpyrrolidone (NMP);
a mass ratio of the triethyl orthoformate to the compound of Formula 5 is in a range of 1:1 to 5:1;
the concentrated sulfuric acid has a mass percentage content of 98%, and a mass ratio of the compound of Formula 5 to the concentrated sulfuric acid is in a range of 1:0.02 to 1:0.3; and
the etherification is conducted at a temperature of 80°C to 130°C.

In some embodiments, in step IV), the tartaric acid derivative is selected from the group consisting of dibenzoyl-L-tartaric acid, L-di(*p*-toluoyl)tartaric acid, dibenzoyl-D-tartaric acid, and D-di(*p*-toluoyl)tartaric acid; a mass ratio of the compound of Formula 6 to the tartaric acid is in a range of 1:0.4 to 1:1.2; the fourth organic solvent is at least one selected from the group consisting of methanol, ethanol, isopropanol, ethyl acetate, n-propyl acetate, isopropyl acetate, butyl acetate, acetone, and butanone; the first temperature is in a range of 30°C to 80°C, and the first heat preservation is conducted for 1 h to 5 h; the second temperature is in a range of 0°C to 25°C, and the second heat preservation is conducted for 1 h to 24 h; and
the fifth organic solvent is at least one selected from the group consisting of ethanol, methanol, isopropanol, n-butanol, and sec-butanol; a volume ratio of the fifth organic solvent to the water is in a range of 1:3 to 1:10; and the basic pH regulator is at least one selected from the group consisting of potassium carbonate, sodium carbonate, sodium bicarbonate, sodium phosphate, and potassium phosphate.

In some embodiments, in step V),
the basic compound includes sodium hydroxide and/or potassium hydroxide, and a mass ratio of the basic compound to the compound of Formula 7 is in a range of 0.2:1 to 2.0:1;
the sixth organic solvent is at least one selected from the group consisting of methanol, ethanol, dichloromethane (DCM), and ethylene glycol dimethyl ether (DME); and
the hydrolysis is conducted at a temperature of 0°C to 30°C.

In some embodiments, in step VI),
the seventh organic solvent is at least one selected from the group consisting of DMF, DMA, NMP, and tetrahydrofuran (THF);
the aqueous ammonia has a mass percentage content of 25% to 28%;
a mass ratio of the compound of Formula 8, the CDI, the DMAP, and the aqueous ammonia is in a range of 1 : 0.3-1.5 : 0.02-0.2 : 3-10; and
the amidation is conducted at a temperature of 70°C to 120°C.

In some embodiments, the method further includes:
in step VI), after conducting the amidation to obtain an amidation reaction mixture, precipitating the amidation reaction mixture with water to obtain a crude product; and subjecting the crude product to purification with an alcoholic solvent to obtain pure finerenone, where the alcoholic solvent is at least one selected from the group consisting of methanol, an aqueous ethanol solution, and isopropanol, and ethanol in the aqueous ethanol solution has a mass percentage content of 95% to 100%; and
in step V), after conducting the hydrolysis to obtain a hydrolysis reaction mixture, neutralizing the hydrolysis reaction mixture with an acid and precipitating a resulting neutralized product with water to obtain the compound of Formula 8, where the acid is at least one selected from the group consisting of hydrochloric acid, sulfuric acid, acetic acid, and formic acid.

The present disclosure further provides finerenone prepared by the method as described above, where the finerenone exhibits an enantiomeric excess (ee) value greater than 99.9%.

The present disclosure further provides a finerenone intermediate prepared by the method as described above, where the finerenone intermediate has a structure selected from the group consisting of the Formula 3, the Formula 5, the Formula 6, and Formula 7:

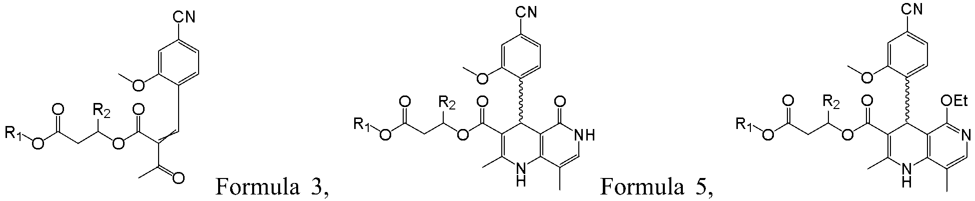

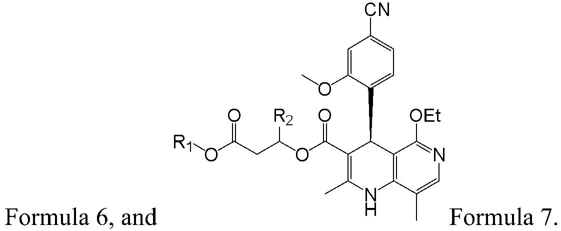

The present disclosure provides a method for preparing finerenone. A compound of Formula 2 having a diester structure is employed as a reaction starting material for a series of reactions. By utilizing a chiral catalyst, a reaction product (namely the compound of Formula 5 and the compound of Formula 6) with an S/R configuration ratio of up to 85:15 is obtained. The product is then resolved by using a tartaric acid derivative to prepare a compound of Formula 7, which is subsequently subjected to hydrolysis and amidation to obtain finerenone. The method enables the production of a finerenone active pharmaceutical ingredient (API) with an enantiomeric excess (ee) value greater than 99.9% and a purity reaching 99.9%. The method offers the following advantages:

The compound of Formula 2, obtained by esterification of 3-hydroxyester with a carboxylic acid, is used as the reaction starting material. After the compound of Formula 2 reacts with the compound of Formula 1 to form the compound of Formula 3, the ester structure derived from the compound of Formula 2 exhibits excellent stability throughout the first 4 reaction steps (Steps I) to IV)). Furthermore, this ester structure can be readily hydrolyzed under basic conditions to easily obtain the required carboxylic acid intermediate (the compound of Formula 8). Furthermore, by utilizing the compound of Formula 2 as the raw material and leveraging the diester structure formed by the compound of Formula 2 in conjunction with a chiral catalyst, the formation of the chiral center in the target product is induced, obtaining the compound of Formula 5 with a significantly higher proportion of the desired configuration relative to the undesired configuration (S:R ratio up to 85:15), and effectively improving the yield of the desired S-configuration product obtained through resolution. Furthermore, all reaction steps in the method involve conventional chemical reactions, avoiding hazardous steps such as hydrogenolysis or high-pressure reactions. The method features mild reaction conditions, absence of highly toxic byproducts, low requirements for reaction equipment and operating costs, and simple operation, making it suitable for industrial production with enhanced market prospects.

### BRIEF DESCRIPTION OF THE DRAWINGS

FIG. 1 shows the H NMR (nuclear magnetic resonance) spectrum of finerenone prepared in Example 6 of the present disclosure;
FIG. 2 shows the C NMR spectrum of finerenone prepared in Example 6 of the present disclosure; and
FIG. 3 shows the HPLC (high performance liquid chromatography) chromatogram of finerenone prepared in Example 6 of the present disclosure.

### DETAILED DESCRIPTION OF THE EMBODIMENTS

The present disclosure provides a method for preparing finerenone, including the following steps:
I) mixing a compound of Formula 1, a compound of Formula 2, an organic base, acetic acid, and a first organic solvent, and conducting condensation, to obtain a compound of Formula 3;
II) mixing the compound of Formula 3, a compound of Formula 4, a chiral catalyst, and a second organic solvent, and conducting cyclization, to obtain a compound of Formula 5;
III) mixing the compound of Formula 5, triethyl orthoformate, concentrated sulfuric acid, and a third organic solvent, and conducting etherification, to obtain a compound of Formula 6;
IV) mixing the compound of Formula 6, a tartaric acid derivative, and a fourth organic solvent, conducting a first heat preservation by maintaining an obtained mixture at a first temperature, and then cooling a resulting material to a second temperature and conducting a second heat preservation, to obtain a salt of the tartaric acid derivative; and mixing the salt of the tartaric acid derivative, water, and a fifth organic solvent, and then regulating a pH value of a resulting mixture to 7.5 to 9.5 by using a basic pH regulator, to obtain a compound of Formula 7;
V) mixing the compound of Formula 7, a sixth organic solvent, and a basic compound, and conducting hydrolysis, to obtain a compound of Formula 8; and
VI) mixing the compound of Formula 8, N,N-carbonyldiimidazole (CDI), a seventh organic solvent, 4-dimethylaminopyridine (DMAP), and aqueous ammonia, and conducting amidation, to obtain the finerenone; 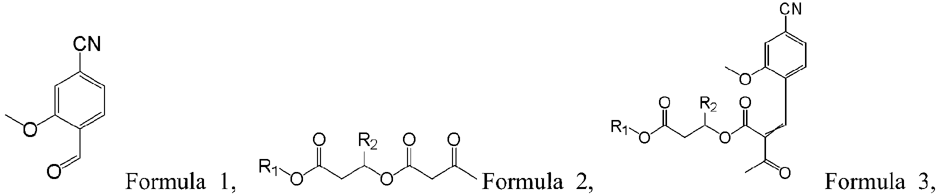 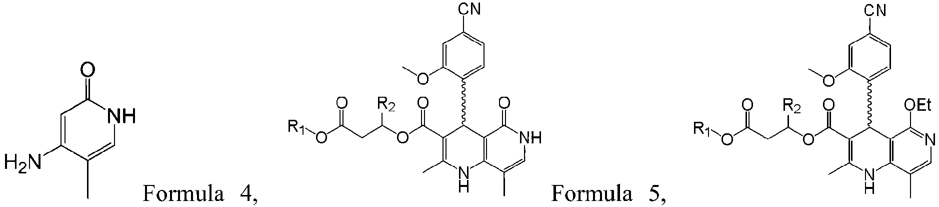 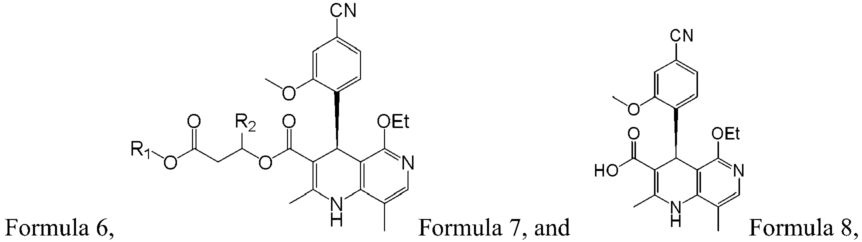 where in Formula 2, Formula 3, Formula 5, Formula 6, and Formula 7, R₁ is C1-10 alkyl, R₂ is selected from the group consisting of hydrogen, C1-10 alkyl, halogen-substituted C1-10 alkyl, and cyano-substituted C1-10 alkyl.

In the present disclosure, unless otherwise specified, all raw materials/components are commercially available products well known to those skilled in the art.

The present disclosure provides a method for preparing finerenone, having a synthetic route as follows:

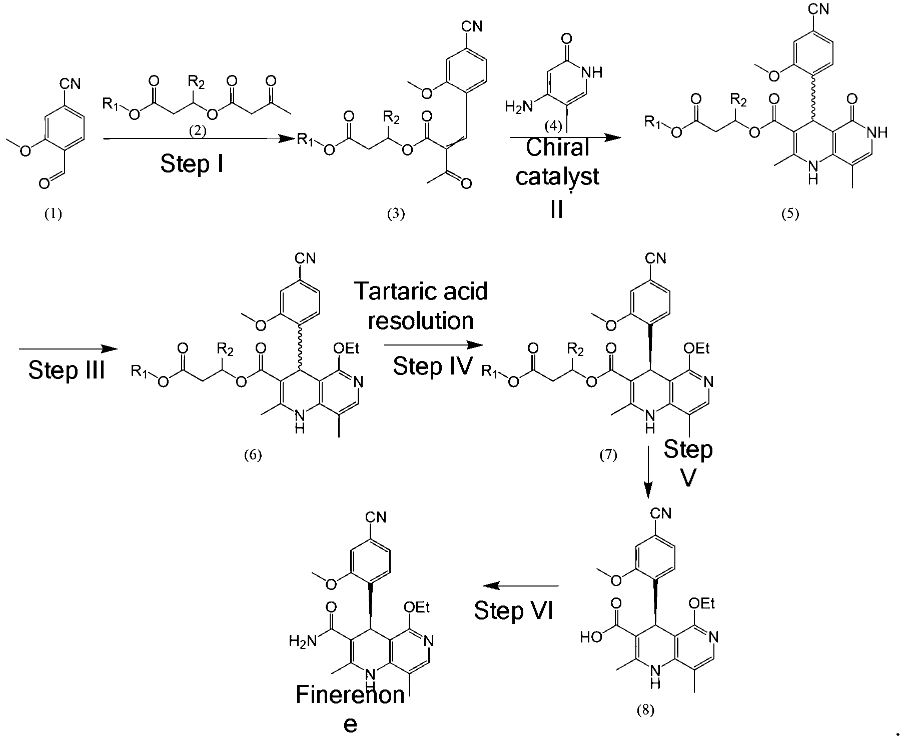

The preparation method provided by the present disclosure is described in detail below in conjunction with the synthetic route of finerenone provided by the present disclosure.

In the present disclosure, a compound of Formula 1, a compound of Formula 2, an organic base, acetic acid, and a first organic solvent are mixed (hereinafter referred to as a first mixing), and a resulting mixture is subjected to condensation to obtain a compound of Formula 3. In some embodiments, in Formula 2: R₁ is C1-8 alkyl, more preferably C1-6 alkyl, specifically methyl, ethyl, n-propyl, isopropyl, n-butyl, or tert-butyl, and most preferably methyl or ethyl. In some embodiments, in R₂: halogen in the halogen-substituted C1-10 alkyl is F, Cl, or Br. In some embodiments, R₂ is hydrogen, C1-5 alkyl, halogen-substituted C1-5 alkyl, or cyano-substituted C1-5 alkyl; further preferably hydrogen, C1-3 alkyl, halogen-substituted C1-3 alkyl, or cyano-substituted C1-3 alkyl; specifically preferably 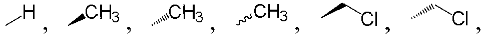 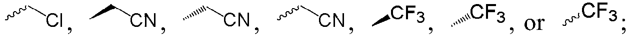 and most preferably 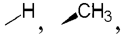 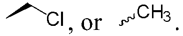 In some embodiments, the organic base includes at least one selected from the group consisting of piperidine, morpholine, and pyrrolidine. In some embodiments, the first organic solvent includes at least one selected from the group consisting of methanol, ethanol, n-propanol, isopropanol, n-butanol, and sec-butanol. In some embodiments, a mass ratio of the compound of Formula 1, the organic base, the acetic acid, and the compound of Formula 2 is in a range of 1:0.03-0.2:0.02-0.2:1.0-2.0, and more preferably 1:0.03-0.05:0.02-0.05:1.0-1.5. In some embodiments, a volume-to-mass ratio of the first organic solvent to the compound of Formula 1 is in a range of 5 mL: 1 g to 20 mL: 1 g, and more preferably 6 mL: 1 g to 15 mL: 1 g. In some embodiments, the first mixing includes the following steps: adding the compound of Formula 1 to the first organic solvent, followed by sequentially adding the acetic acid and the organic base to obtain a mixture; slowly dropwise adding the compound of Formula 2 to the mixture. In some embodiments, the condensation is conducted at a temperature of 20°C to 50°C, and more preferably 25°C to 45°C. In some embodiments, the condensation is conducted until complete conversion of the starting material. In some embodiments, after completion of the condensation, a solvent is removed from a resulting condensation reaction mixture to obtain the compound of Formula 3. In some embodiments, removing the solvent is conducted by concentration.

After obtaining the compound of Formula 3, the compound of Formula 3, a compound of Formula 4, a chiral catalyst, and a second organic solvent are mixed (hereinafter referred to as the second mixing), and a resulting mixture is subjected to cyclization, to obtain a compound of Formula 5. In some embodiments, the chiral catalyst includes D-(+)-10-camphorsulfonic acid or (S)-(+)-1,1'-binaphthyl-2,2'-diyl hydrogenphosphate. In some embodiments, the second organic solvent includes at least one selected from the group consisting of isopropanol, tert-butanol, sec-butanol, and sec-amyl alcohol. In some embodiments, a mass ratio of the compound of Formula 3, the compound of Formula 4, and the chiral catalyst is in a range of 1:0.2-1.2:0.2-2.0, and more preferably 1:0.2-0.4:0.2-0.4. In some embodiments, a volume-to-mass ratio of the second organic solvent and the compound of Formula 3 is in a range of 5 mL:1 g to 20 mL:1 g, and more preferably 5.2 mL:1 g to 18 mL: 1 g. In some embodiments, the second mixing includes the following steps: sequentially adding the compound of Formula 3, the compound of Formula 4, and the chiral catalyst to the second organic solvent. In some embodiments, the cyclization is conducted at a temperature of 70°C to 120°C under heating reflux conditions. In some embodiments, the cyclization is conducted under stirring conditions. In some embodiments, the cyclization is conducted until complete conversion of the starting material. In some embodiments, after completion of the cyclization, a cyclization reaction mixture is obtained. In some embodiments, the cyclization reaction mixture is cooled to room temperature, and subjected to solid-liquid separation to obtain a solid product. In some embodiments, the solid product is dried to obtain the compound of Formula 5. In some embodiments, the solid-liquid separation is filtration, and drying is conducted by oven drying.

After obtaining the compound of Formula 5, the compound of Formula 5, triethyl orthoformate, concentrated sulfuric acid, and a third organic solvent are mixed (hereinafter referred to as a third mixing), and a resulting mixture is subjected to etherification, to obtain a compound of Formula 6. In some embodiments, the concentrated sulfuric acid has a mass percentage content of 98%. In some embodiments, the third organic solvent includes at least one selected from the group consisting of DMF, DMA, and NMP. In some embodiments, a mass ratio of the triethyl orthoformate to the compound of Formula 5 is in a range of 1:1 to 5:1, and more preferably 2:1 to 3:1. In some embodiments, a mass ratio of the compound of Formula 5 to the concentrated sulfuric acid is in a range of 1:0.02 to 1:0.3, and more preferably 1:0.1 to 1:0.2. In some embodiments, a volume-to-mass ratio of the third organic solvent to the compound of Formula 5 is in a range of 2 mL:1 g to 10 mL:1 g, and more preferably 2.5 mL: 1 g to 9 mL:1 g. In some embodiments, the third mixing includes the following steps: sequentially adding the compound of Formula 5 and the triethyl orthoformate to the third organic solvent, controlling a temperature at 100°C, and adding the concentrated sulfuric acid thereto. In some embodiments, the etherification is conducted at a temperature of 80°C to 130°C, and more preferably 100°C. In some embodiments, the etherification is conducted until complete conversion of the starting material. In some embodiments, after completion of the etherification, an etherification reaction mixture is obtained. In some embodiments, the etherification reaction mixture is subjected to cooling, precipitating with water, solid-liquid separation, and drying in sequence to obtain the compound of Formula 6. In some embodiments, the cooling is conducted to room temperature, and a solid product obtained from the solid-liquid separation is dried. In some embodiments, the solid-liquid separation is filtration, and the drying is oven drying.

After obtaining the compound of Formula 6, the compound of Formula 6, a tartaric acid derivative, and a fourth organic solvent are mixed (hereinafter referred to as the fourth mixing); a resulting mixture is subjected to first heat preservation at a first temperature, then a resulting heated product is cooled to a second temperature and subjected to second heat preservation, to obtain a salt of the tartaric acid derivative; and the salt of the tartaric acid derivative, water, and a fifth organic solvent are then mixed (hereinafter referred to as a fifth mixing), and a pH value of an obtained mixture is regulated to 7.5 to 9.5 with a basic pH regulator to obtain a compound of Formula 7. In some embodiments, the tartaric acid derivative includes dibenzoyl-L-tartaric acid, L-di(*p*-toluoyl)tartaric acid, dibenzoyl-D-tartaric acid, or D-di(*p*-toluoyl)tartaric acid. In some embodiments, a mass ratio of the compound of Formula 6 to the tartaric acid derivative is in a range of 1:0.4 to 1:1.2, and more preferably 1:0.5 to 1:0.8. In some embodiments, the fourth organic solvent includes at least one selected from the group consisting of methanol, ethanol, isopropanol, ethyl acetate, n-propyl acetate, isopropyl acetate, butyl acetate, acetone, and butanone; more preferably one or two selected from the group consisting of methanol, ethanol, isopropanol, ethyl acetate, n-propyl acetate, isopropyl acetate, butyl acetate, acetone, and butanone. In some embodiments, under a condition that the fourth organic solvent includes any two of the aforementioned substances, a volume ratio of any two solvents is 1:1. In a specific embodiment, the fourth organic solvent includes ethyl acetate and/or methanol. In some embodiments, a volume-to-mass ratio of the fourth organic solvent to the compound of Formula 6 is in a range of 10 mL:1 g to 30 mL:1 g, and more preferably 12 mL:1 g to 26 mL:1 g. In some embodiments, the fourth mixing includes the following step: adding the compound of Formula 6 and the tartaric acid derivative to the fourth organic solvent. In some embodiments, the first temperature is in a range of 30°C to 80°C, and more preferably 70°C. The first heat preservation is conducted for preferably 1 h to 5 h, and more preferably 2 h. In some embodiments, the first heat preservation is conducted under stirring conditions. In some embodiments, the second temperature is in a range of 0°C to 25°C, and more preferably 15°C to 23°C; the second heat preservation is conducted for 1 h to 24 h, and more preferably 8 h. In some embodiments, the second heat preservation is conducted under stirring conditions. In some embodiments, after the second heat preservation is completed, a resulting reaction mixture is subjected to solid-liquid separation to obtain the salt of the tartaric acid derivative. In some embodiments, the solid-liquid separation is filtration.

After obtaining the salt of the tartaric acid derivative, the salt of the tartaric acid derivative, water, and the fifth organic solvent are subjected to the fifth mixing; a pH value of a resulting mixture is regulated to 7.5 to 9.5 with a basic pH regulator to obtain the compound of Formula 7. In some embodiments, the fifth organic solvent includes at least one selected from the group consisting of ethanol, methanol, isopropanol, n-butanol, and sec-butanol. In some embodiments, a volume ratio of the fifth organic solvent to the water is in a range of 1:3 to 1:10, and more preferably 1:4 to 1:8. In some embodiments, a volume-to-mass ratio of the fifth organic solvent to the compound of Formula 6 is in a range of 10 mL:1 g to 30 mL:1 g, and more preferably 11 mL:1 g to 28 mL:1 g. In some embodiments, the fifth mixing includes the following step: mixing the water and the fifth organic solvent, and then adding the salt of the tartaric acid derivative thereto. In some embodiments, the basic pH regulator includes at least one selected from the group consisting of potassium carbonate, sodium carbonate, sodium bicarbonate, sodium phosphate, and potassium phosphate. In some embodiments, the pH value is regulated to 7.5. In some embodiments, after regulating the pH value to 7.5 to 9.5, a resulting reaction mixture is subjected to solid-liquid separation, and an obtained solid product is dried to obtain the compound of Formula 7. In some embodiments, the solid-liquid separation is conducted by filtration. In some embodiments, drying is conducted by oven drying.

After obtaining the compound of Formula 7, the compound of Formula 7, a sixth organic solvent, and a basic compound are mixed (hereinafter referred to as a sixth mixing), and a resulting mixture is subjected to hydrolysis, to obtain a compound of Formula 8. In some embodiments, the basic compound includes sodium hydroxide and/or potassium hydroxide. In some embodiments, the sixth organic solvent includes at least one selected from the group consisting of methanol, ethanol, DCM, and DME; more preferably one or two selected from the group consisting of methanol, ethanol, DCM, and DME. In some embodiments, under a condition that the sixth organic solvent includes any two of the aforementioned substances, a volume ratio of any two solvents is 1:1. In a specific embodiment, the sixth organic solvent includes methanol and/or DCM. In some embodiments, a mass ratio of the basic compound to the compound of Formula 7 is in a range of 0.2:1 to 2.0:1, and preferably 0.3:1 to 1.5:1. In some embodiments, a volume-to-mass ratio of the sixth organic solvent to the compound of Formula 7 is in a range of 5 mL:1 g to 30 mL:1 g, and preferably 6 mL:1 g to 25 mL:1 g. In some embodiments, the sixth mixing includes the following steps: adding the compound of Formula 7 to the sixth organic solvent, controlling a temperature at 0°C to 30°C, and adding the basic compound thereto. In some embodiments, the hydrolysis is conducted at a temperature of 0°C to 30°C, and more preferably 5°C to 10°C. In some embodiments, the hydrolysis is conducted under stirring conditions. In some embodiments, the reaction is conducted until complete conversion of the starting material. In some embodiments, after completion of the hydrolysis, a hydrolysis reaction mixture is obtained. In some embodiments, the method further includes: neutralizing the hydrolysis reaction mixture with an acid, followed by precipitating with water, to obtain the compound of Formula 8; where the acid includes at least one selected from the group consisting of hydrochloric acid, sulfuric acid, acetic acid, and formic acid, and more preferably hydrochloric acid.

After obtaining the compound of Formula 8, the compound of Formula 8, CDI, a seventh organic solvent, DMAP, and aqueous ammonia are mixed (hereinafter referred to as a seventh mixing), and a resulting mixture is subjected to amidation, to obtain the finerenone. In some embodiments, the seventh organic solvent includes at least one selected from the group consisting of DMF, DMA, NMP, and THF. In some embodiments, the aqueous ammonia has a mass percentage content of 25% to 28%. In some embodiments, a mass ratio of the compound of Formula 8, the CDI, the DMAP, and the aqueous ammonia is in a range of 1:0.3-1.5:0.02-0.2:3-10, and more preferably 1:0.35-1.4:0.03-0.16:3.5-8. In some embodiments, a volume-to-mass ratio of the seventh organic solvent to the compound of Formula 8 is in a range of 3 mL:1 g to 10 mL:1 g, and more preferably 3.5 mL:1 g to 8 mL:1 g. In some embodiments, the seventh mixing includes the following steps: adding the compound of Formula 8 and CDI to the seventh organic solvent, stirring a resulting mixture at room temperature for 2 h, then adding the DMAP and aqueous ammonia thereto. In some embodiments, the amidation is conducted at a temperature of 70°C to 120°C, and more preferably 80°C to 100°C. In some embodiments, the amidation is conducted under stirring conditions. In some embodiments, the amidation is conducted until complete conversion of the starting material. In some embodiments, an amidation reaction mixture is obtained after the amidation. In some embodiments, the method further includes: precipitating the amidation reaction mixture with water to obtain a crude product; subjecting the crude product to purification with an alcoholic solvent to obtain pure finerenone; where the alcoholic solvent includes at least one selected from the group consisting of methanol, an aqueous ethanol solution, and isopropanol, and ethanol in the aqueous ethanol solution has a mass percentage content of 95% to 100%. In some embodiments, the purification specifically includes: dissolving the crude product in the alcoholic solvent by heating to obtain a crude product solution, and then subjecting the crude product solution to concentration, cooling crystallization, solid-liquid separation, and drying in sequence, thereby obtaining the pure finerenone. In some embodiments, a volume ratio of a concentrated solution obtained after the concentration to the crude product solution is in a range of 1:5 to 1:7. In some embodiments, the cooling crystallization is conducted at an initial temperature of 50°C to 60°C, an endpoint temperature of -5°C to 5°C, and a cooling rate of 1°C/min to 3°C/min. In some embodiments, the solid-liquid separation is conducted by filtration.

The present disclosure further provides finerenone prepared by the method as described above, where the finerenone exhibits an enantiomeric excess (ee) value greater than 99.9%.

The present disclosure further provides a finerenone intermediate prepared by the method as described above, where the finerenone intermediate has a structure selected from the group consisting of the Formula 3, the Formula 5, the Formula 6, and the Formula 7:

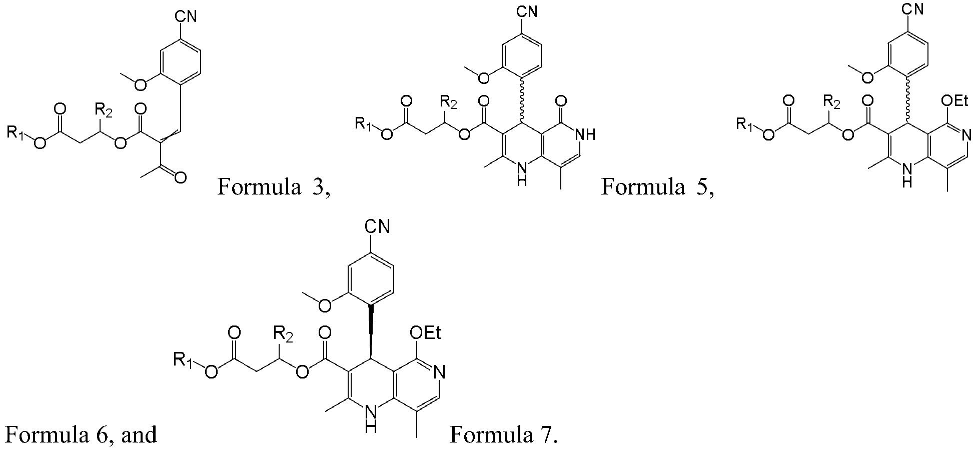

In order to further illustrate the present disclosure, the technical solutions provided by the present disclosure are described in detail below in connection with examples, but these examples should not be understood as limiting the scope of the present disclosure.

Specific experimental procedures or conditions that are not indicated in the present disclosure shall be implemented by operations of conventional experimental procedures described in the disclosure in the art. All used reagents or equipment without specifying manufacturers may be commercially available conventional products.

### Example 1

### Preparation of (R)-4-ethoxy-4-oxobutan-2-yl-2-(4-cyano-2-methoxybenzylidene)-3-oxobutanoate (structure as follows):

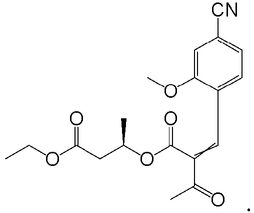

50 g of 4-formyl-3-methoxybenzonitrile were added to 500 mL of isopropanol. 1 g of acetic acid and 1.4 g of piperidine were added thereto. A temperature was controlled at 30°C, and 75 g of (R)-4-ethoxy-4-oxobutan-2-yl-3-oxobutanoate were slowly added dropwise. A resulting mixture was reacted by maintaining at the temperature until reaction was complete. A solvent was then concentrated off, obtaining 110.9 g of titled compound, with a yield of 99.5%.

### Example 2

### Preparation of (R)-4-ethoxy-4-oxobutan-2-yl-4-(4-cyano-2-methoxyphenyl)-2,8-dimethyl-5-oxo-1,4,5,6-tetrahydro-1,6-naphthyridine-3-carboxylate (structure as follows):

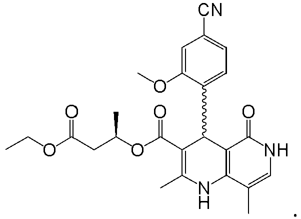

110.9 g of (R)-4-ethoxy-4-oxobutan-2-yl-2-(4-cyano-2-methoxybenzylidene)-3-oxobutanoate and 35 g of a compound of Formula 4 (4-amino-5-methylpyridone) were added to 600 mL of 2-butanol. 30 g of (S)-(+)-1,1'-binaphthyl-2,2'-diyl hydrogenphosphate were added thereto. A resulting mixture was stirred and heated under reflux for 24 h, and a resulting heated product was cooled to room temperature, filtered, and then oven-dried to obtain 123.6 g of titled compound (S:R ratio = 85:15, desired configuration: S-form), with a yield of 94.1 % (based on the 4-amino-5-methylpyridone).

### Example 3

### Preparation of (R)-4-ethoxy-4-oxobutan-2-yl-4-(4-cyano-2-methoxyphenyl)-5-ethoxy-2,8-dimethyl-1,4-dihydro-1,6-naphthyridine-3-carboxylate (structure as follows):

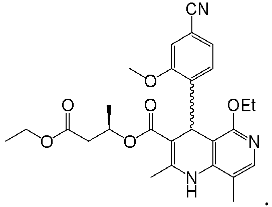

123.6 g of (R)-4-ethoxy-4-oxobutan-2-yl-4-(4-cyano-2-methoxyphenyl)-2,8-dimethyl-5-oxo-1,4,5,6-tetrahydro-1,6-naphthyridine-3-carboxylate (from Example 2) and 350 g of triethyl orthoformate were added to 450 mL of DMA. A temperature was maintained at 100°C, and 11 g of sulfuric acid (98 wt%) were slowly added thereto. A resulting mixture was held at the temperature until raw materials completed conversion. A resulting product was subjected to cooling, water precipitation, filtration, and oven-drying in sequence to obtain 123.5 g of titled compound (S:R ratio = 85:15, desired configuration: S-form), with a yield of 94.2%.

### Example 4

### Preparation of (R)-4-ethoxy-4-oxobutan-2-yl (4S)-4-(4-cyano-2-methoxyphenyl)-5-ethoxy-2,8-dimethyl-1,4-dihydro-1,6-naphthyridine-3-carboxylate (structure as follows):

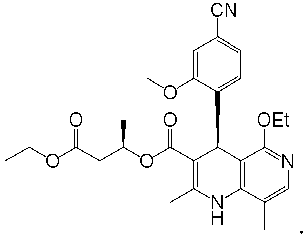

100 g of (R)-4-ethoxy-4-oxobutan-2-yl-4-(4-cyano-2-methoxyphenyl)-5-ethoxy-2,8-dimethyl-1,4-dihydro-1,6-naphthyridine-3-carboxylate (from Example 3) and 75 g of dibenzoyl-D-tartaric acid were added to a mixture of 600 mL of ethyl acetate and 600 mL of methanol. A resulting mixture was stirred at 70°C for 2 h, cooled to 20°C, and stirred for 8 h. A resulting product was subjected to filtration, an obtained solid was added to 1200 mL of a 20 vol% aqueous isopropanol solution. A pH value of a resulting mixture was regulated to 7.5 by using an aqueous sodium phosphate solution. A resulting mixture was filtered and oven-dried to obtain 84.5 g of titled compound (ee value = 99.3%), with a yield of 84.5%.

### Example 5

### Preparation of (S)-4-(4-cyano-2-methoxyphenyl)-5-ethoxy-2,8-dimethyl-1,4-dihydro-1,6-naphthyridine-3-carboxylic acid (structure as follows):

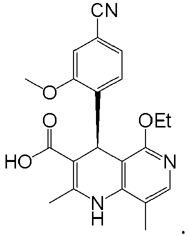

84.5 g of (R)-4-ethoxy-4-oxobutan-2-yl (4S)-4-(4-cyano-2-methoxyphenyl)-5-ethoxy-2,8-dimethyl-1,4-dihydro-1,6-naphthyridine-3-carboxylate (from Example 4) were added to a mixture of 350 mL of methanol and 350 mL of DCM. At 5°C, 25 g of potassium hydroxide were added thereto. A resulting mixture was stirred until raw materials completed conversion, and a resulting product was neutralized with hydrochloric acid, water precipitated, filtered, and oven-dried to obtain 63.3 g of titled compound, with a yield of 97.4%.

### Example 6

### Preparation of (S)-4-(4-cyano-2-methoxyphenyl)-5-ethoxy-2,8-dimethyl-1,4-dihydro-1,6-naphthyridine-3-carboxamide (structure as follows):

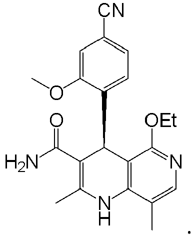

63.3 g of (S)-4-(4-cyano-2-methoxyphenyl)-5-ethoxy-2,8-dimethyl-1,4-dihydro-1,6-naphthyridine-3-carboxylic acid and 35 g of CDI were added to 270 mL of DMF. A resulting mixture was stirred at room temperature for 2 h, 3.5 g of DMAP and 270 g of aqueous ammonia (28 wt%) were added thereto. A resulting mixture was subjected to reaction at 80°C until completion. A resulting reaction product was subjected to cooling and water precipitation, filtration in sequence to obtain a wet cake, and the wet cake was purified with isopropanol. Purification was conducted by dissolving the wet cake in 1000 mL of isopropanol under heating, and a resulting mixture was concentrated to obtain approximately 200 mL of a concentrated solution. The concentrated solution was cooled from 60°C to 5°C at 2°C/min (no holding period), then filtered. A resulting solid product was dried to obtain 57.5 g of finerenone (ee value = 100%, HPLC purity = 99.9%), with a yield of 90.9%.

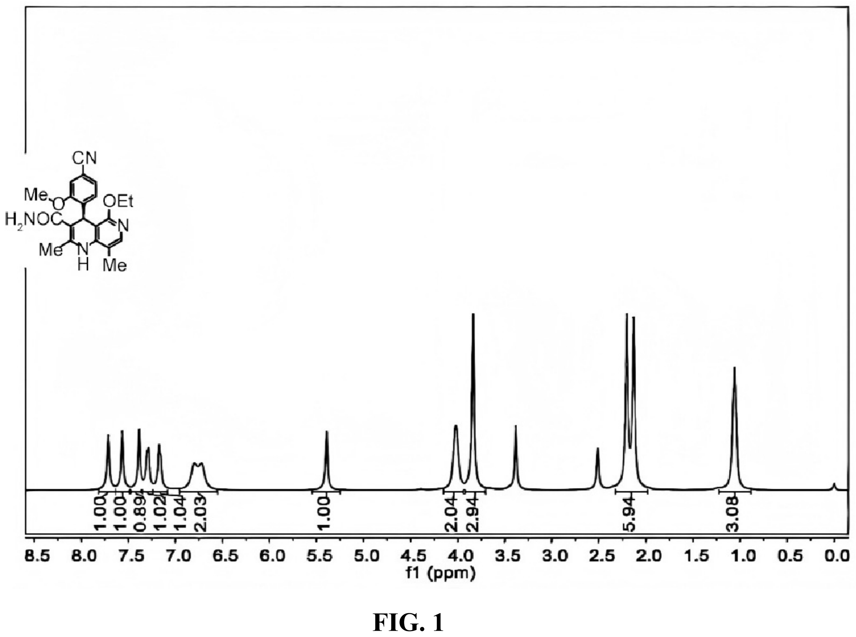

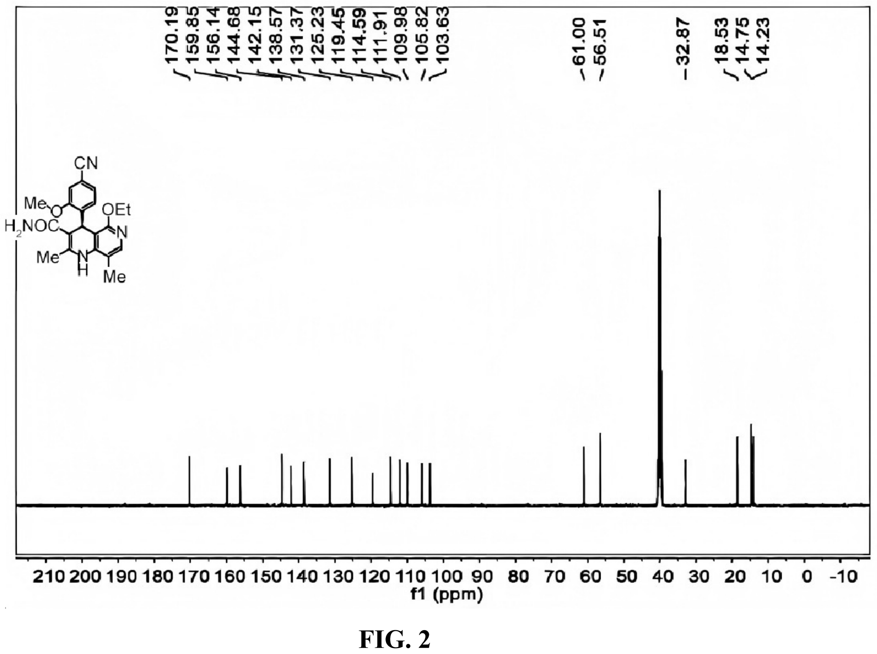

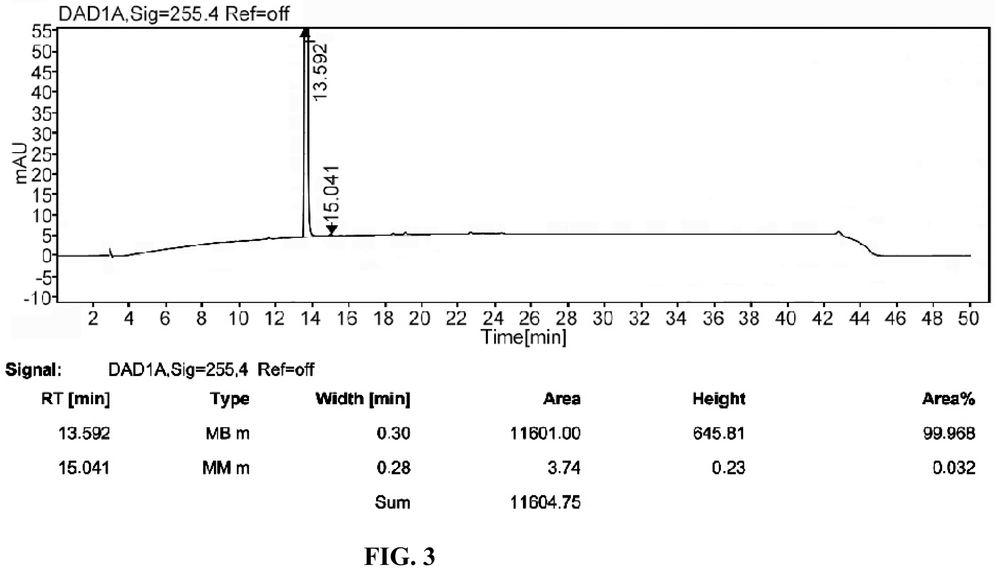

The H NMR spectrum of finerenone prepared in Example 6 is shown in FIG. 1; the C NMR spectrum of finerenone prepared in Example 6 is shown in FIG. 2; and the HPLC chromatogram of finerenone prepared in Example 6 is shown in FIG. 3.

### Example 7

The preparation process for finerenone and its intermediates in this example was as follows:

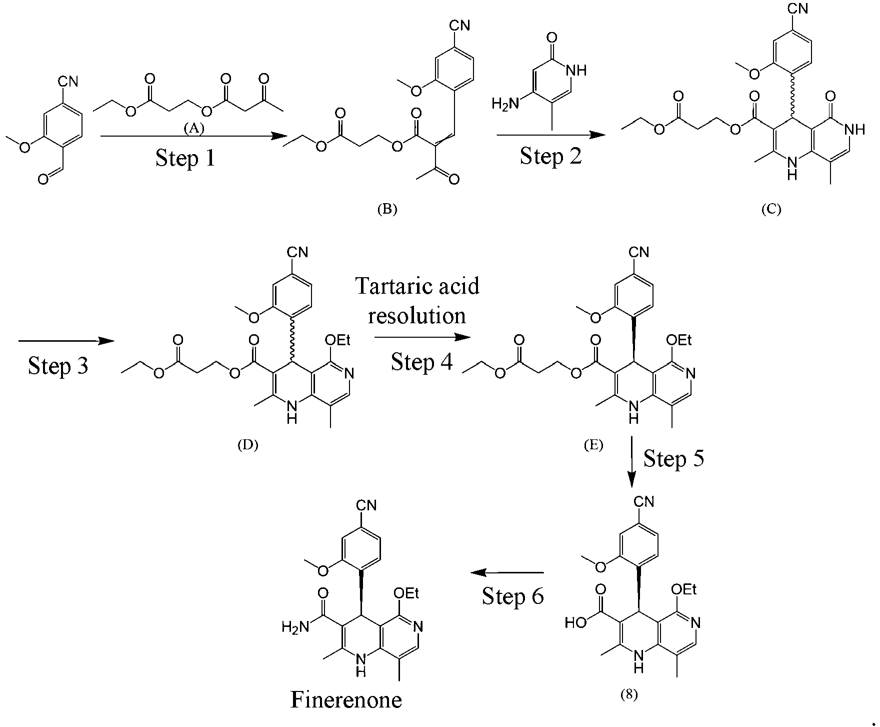

Step 1: 50 g of 4-cyano-2-methoxybenzaldehyde was added to 250 mL of isopropanol. 1.2 g of acetic acid and 1.69 g of piperidine were added thereto. A temperature was controlled at 20°C, and 65 g of (3-ethoxy-3-oxopropyl) 3-oxobutanoate was slowly added dropwise thereto for reaction. A resulting mixture was held at the temperature until the reaction was complete. A solvent was concentrated off, obtaining 105.3 g of compound (B), with a yield of 98.4%.

Step 2: 105.3 g of the compound (B) obtained in Step 1 and 37 g of 4-amino-5-methylpyridone were added to 1000 mL of 2-butanol. 25 g of (S)-(+)-1,1'-binaphthyl-2,2'-diyl hydrogenphosphate was added thereto. A resulting mixture was stirred and heated under reflux for 24 h, and a resulting heated product was cooled to room temperature, filtered, and oven-dried to obtain 124.1 g of compound (C) (S:R configuration ratio = 75:25), with a yield of 92.1%.

Step 3: 124.1 g of the compound (C) obtained in Step 2 and 180 g of triethyl orthoformate were added to 600 mL of DMA. A temperature was maintained at 80°C, and 9.2 g of sulfuric acid (98 wt%) was slowly added thereto. A resulting mixture was held at the temperature until raw materials completed conversion. A resulting product was subjected to cooling, water precipitation, filtration, and oven-drying, and 123.3 g of compound (D) (S:R ratio = 75:25) was obtained, with a yield of 93.5%.

Step 4: 100 g of compound (D) obtained in Step 3 and 80 g of L-di(*p*-toluoyl)tartaric acid were added to 1200 mL of n-propyl acetate. A resulting mixture was stirred at 60°C for 4 h, cooled to 0°C, and stirred for 12 h. A resulting mixture was subjected to filtration, an obtained solid was added to 1,200 mL of a 30 vol% aqueous ethanol solution. A pH value was regulated to 7.5 by using an aqueous potassium carbonate solution. A resulting mixture was filtered and oven-dried to obtain 74.3 g of compound (E) (ee value = 99.5%), with a yield of 74.3%.

Step 5: 74.3 g of the compound (E) obtained in Step 4 was added to 600 mL of methanol. At 15°C, 16 g of sodium hydroxide was added thereto. A resulting mixture was stirred until raw materials complete conversion, and a resulting product was neutralized with concentrated hydrochloric acid, water precipitated, filtered, and oven-dried to obtain 57.3 g of compound (8), with a yield of 97.4%.

Step 6: 57.3 g of the compound (8) obtained in Step 5 and 34 g of CDI were added to 200 mL of THF. A resulting mixture was stirred at room temperature for 2 h, 4 g of DMAP and 200 g of aqueous ammonia (28 wt%) were added thereto for reaction. The reaction was conducted at 70°C until completion. A resulting reaction product was subjected to cooling and water precipitation, and filtration in sequence to obtain a wet cake, and the wet cake was purified with ethanol. Purification was conducted by dissolving the wet cake in 900 mL of ethanol under heating, and a resulting mixture was concentrated to obtain approximately 150 mL of a concentrated solution. The concentrated solution was cooled from 55°C to 0°C at 3°C/min (no holding period), then filtered. A resulting solid product was dried to obtain 51.7 g of finerenone (ee value = 100%, HPLC purity = 99.7%), with a yield of 90.2%.

### Example 8

The preparation process for finerenone and its intermediates in this example was as follows:

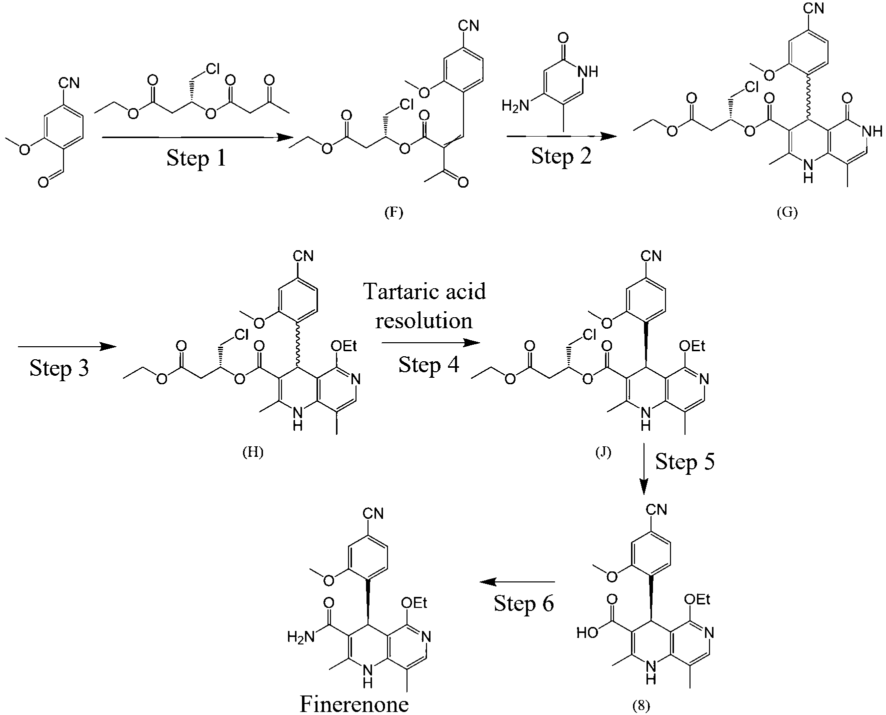

Step 1: 50 g of 4-cyano-2-methoxybenzaldehyde was added to 350 mL of anhydrous ethanol. 1.5 g of acetic acid and 1.8 g of pyrrolidine were added thereto. A temperature was controlled at 40°C, and 75 g of methyl 4-chloro-3-(3-oxobutanoyloxy)butanoate was slowly added dropwise thereto for reaction. A resulting mixture was held at the temperature until the reaction was complete. A solvent was concentrated off, obtaining 119.3 g of compound (F), with a yield of 97.7%.

Step 2: 119.3 g of the compound (F) obtained in Step 1 and 37 g of 4-amino-5-methylpyridone were added to 800 mL of isopropanol. 45 g of D-(+)-10-camphorsulfonic acid was added thereto. A resulting mixture was stirred and heated under reflux for 24 h, and a resulting heated product was cooled to room temperature, filtered, and oven-dried to obtain 135 g of compound (G) (S:R configuration ratio = 78:22), with a yield of 90.5%.

Step 3: 135 g of the compound (G) obtained in Step 2 and 225 g of triethyl orthoformate were added to 500 mL of DMA. A temperature was maintained at 100°C, and 12.9 g of sulfuric acid (98 wt%) was slowly added thereto. A resulting mixture was held at the temperature until raw materials completed conversion. A resulting reaction product was subjected to cooling, water precipitation, filtration, and oven-drying in sequence, to obtain 128.5 g of compound (H) (S:R ratio = 78:22), with a yield of 90.1%.

Step 4: 100 g of the compound (H) obtained in Step 3 and 90 g of L-di(*p*-toluoyl)tartaric acid were added to 1,500 mL of isopropyl acetate. A resulting mixture was stirred at 70°C for 4 h, cooled to 0°C, and stirred for 12 h. A resulting mixture was subjected to filtration, and an obtained solid was added to 1,500 mL of a 25 vol% aqueous methanol solution. A pH value was regulated to 7.5 by using an aqueous sodium bicarbonate solution. A resulting mixture was filtered and oven-dried to obtain 76.5 g of compound (J) (ee value = 99.1%), with a yield of 76.5%.

Step 5: 76.5 g of the compound (J) obtained in Step 4 was added to 850 mL of ethanol. At 5°C, 15 g of potassium hydroxide was added thereto. A resulting mixture was stirred until raw materials completed conversion, and a resulting product was neutralized with formic acid, water precipitated, filtered, and oven-dried to obtain 52.2 g of compound (8), with a yield of 95.0%.

Step 6: 52.2 g of the compound (8) obtained in Step 5 and 35 g of CDI were added to 250 mL of NMP. A resulting mixture was stirred at room temperature for 2 h, 5 g of DMAP and 200 g of aqueous ammonia (28 wt%) were added thereto for reaction. The reaction was conducted at 100°C until completion. A resulting reaction product was subjected to cooling and water precipitation, and filtration in sequence to obtain a wet cake, and the wet cake was purified with a 95 wt% aqueous ethanol solution. Purification was conducted by dissolving the wet cake in 1,200 mL of 95 wt% aqueous ethanol solution under heating, and a resulting mixture was concentrated to obtain approximately 180 mL of a concentrated solution. The concentrated solution was cooled from 60°C to -5°C at 1°C/min (no holding period), then filtered. A resulting solid product was dried to obtain 46.9 g of finerenone (ee value = 100%, HPLC purity = 99.8%), with a yield of 90.0%.

### Example 9

The preparation process for finerenone and its intermediates in this example was as follows:

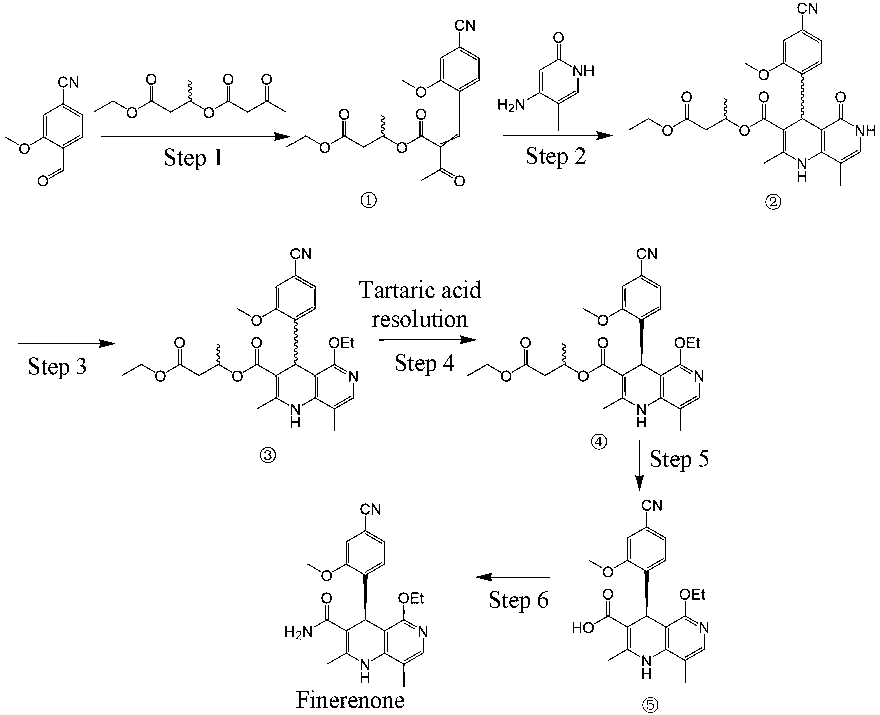

Step 1: following the experimental method of Example 1, except that (R)-4-ethoxy-4-oxobutan-2-yl-3-oxobutanoate was replaced with racemic 4-ethoxy-4-oxobutan-2-yl-3-oxobutanoate. A target product compound ① was obtained with a yield of 99.0%.

Step 2: following the experimental method of Example 2, a target product compound ② (S:R ratio = 80:20) was obtained with a yield of 93.5%.

Step 3: following the experimental method of Example 3, a target product compound ③ (S:R ratio = 80:20) was obtained with a yield of 94.5%.

Step 4: following the experimental method of Example 4, a target product compound ④ (ee value = 99.5%) was obtained with a yield of 79.5%.

Step 5: following the experimental method of Example 5, a target product compound ⑤ was obtained with a yield of 97.5%.

Step 6: following the experimental method of Example 6, finerenone (ee value = 100%, HPLC purity = 99.6%) was obtained with a yield of 90.3%

### Comparative Example 1

The preparation process for finerenone and its intermediates according to the prior art route in this comparative example was as follows:

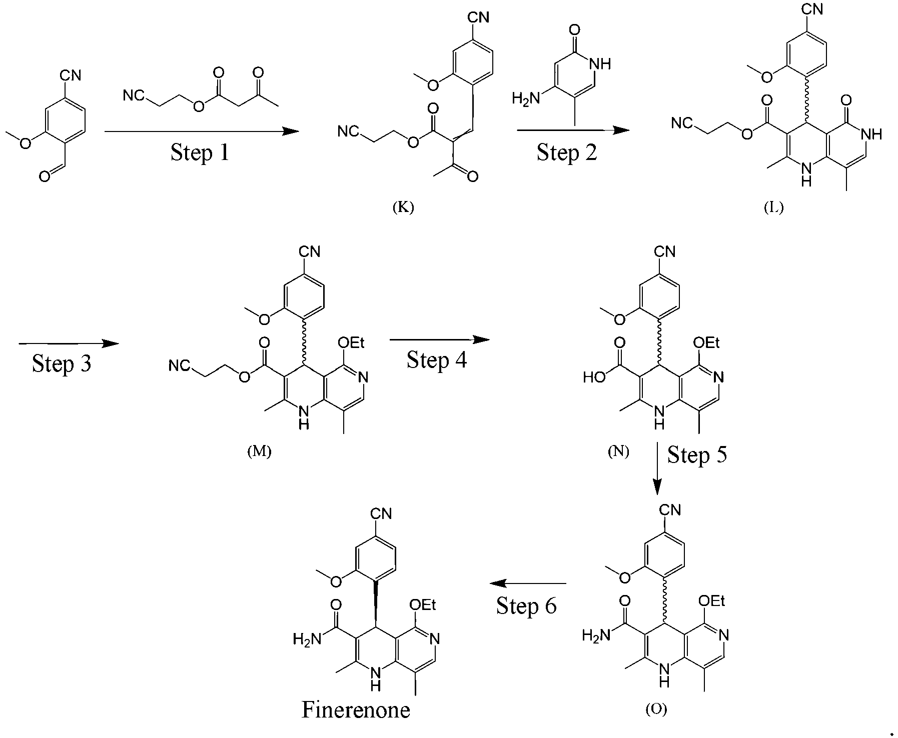

Step 1: 50 g of 4-cyano-2-methoxybenzaldehyde was added to 223 mL of isopropanol. 1.86 mL of acetic acid and 2.64 g of piperidine were added thereto. A temperature was controlled at 30°C, and a solution of 65 g of 3-oxobutanoic acid 2-cyanoethyl ester in 25 mL of isopropanol was slowly added dropwise thereto. A resulting mixture was maintained for 1 h, cooled to 0°C, filtered, and dried to obtain 89.7 g of compound (K), with a yield of 96.8%.

Step 2: 89.7 g of the compound (K) obtained in Step 1 and 36.5 g of 4-amino-5-methylpyridone were added to 1,078 mL of isopropanol. A resulting mixture was stirred under heating reflux at an internal temperature of 100°C under 1.4 bar pressure for 24 h, and a resulting product was cooled to 0°C, filtered, and oven-dried to obtain 104.8 g of compound (L) (S/R configuration ratio = 1:1), with a yield of 88.0%.

Step 3: 104.8 g of compound (L) and 229.8 g of triethyl orthoacetate were dissolved in 594 mL of DMA, and 7.7 g of concentrated sulfuric acid (98 wt%) was added thereto. A resulting mixture was heated at 115°C for 1.5 h, then cooled to 50°C. At 50°C, 1,188 mL of water was added thereto dropwise over 30 min. A resulting mixture was cooled to 0°C (gradient, 2 h) and stirred at 0°C for 2 h. A resulting product was filtered off, washed twice with 375 mL of water each time, and dried under vacuum at 50°C to obtain 103.4 g of compound (M) (desired/undesired configuration ratio = 1:1), with a yield of 92.2%.

Step 4: 100 g of compound (M) was dissolved in 600 mL of THF and 300 mL of water and cooled to 0°C. An aqueous sodium hydroxide solution (prepared from 41 g of 45% NaOH aqueous solution and 211 mL of water) was added thereto dropwise over 15 min, then a resulting mixture was stirred at 0°C for 1.5 h. A resulting mixture was extracted twice with 240 mL of methyl tert-butyl ether each time and once with 240 mL of ethyl acetate. A resulting aqueous solution was adjusted to pH=7 at 0°C with dilute hydrochloric acid (prepared from 18.5 g of 37% hydrochloric acid and 75.5 mL of water). A resulting solution was warmed to 20°C, and an aqueous solution of 102.5 g of ammonium chloride in 277 mL of water was added thereto. A resulting mixed solution was stirred at 20°C for 1 h. A resulting product was filtered off, washed twice with 75 mL of water each time and once with 200 mL of acetonitrile, and dried at 40°C to obtain 86.8 g of compound (N) ,with a yield of 99%.

Step 5: 80 g of compound (N) and 47.9 g of 1,1-carbonyldiimidazole were added to 400 mL of THF, and 2.55 g of DMAP was added thereto at 20°C. A resulting mixture was stirred at 20°C for 1 h, then heated to 50°C for 2.5 h. 148.6 g of hexamethyldisilazane was added to a resulting solution, and a resulting mixture was boiled under reflux for 2 h. An additional 90 mL of THF was added thereto, and a resulting mixture was cooled to 5°C. A mixture of 58.5 mL of THF and 42 g of water was added thereto over 3 h, maintaining a temperature of 5°C to 20°C. A resulting mixture was then boiled under reflux for 1 h, and a resulting product was then cooled to 0°C by gradient (3 h) and stirred at 0°C for 1 h. A resulting product was filtered off, washed twice with 120 mL of THF each time and twice with 160 mL of water each time, and dried under vacuum at 70°C to obtain 75 g of compound (O), with a yield of 94%.

Step 6: 75 g of compound (O) was dissolved in 1.5 L of a mixture of methanol/acetonitrile (60:40, v/v) and subjected to chromatographic separation by using SMB (simulated moving bed). After concentrating a resulting product-containing fractions, 36 g of finerenone in acetonitrile/methanol (40:60, v/v) solution was obtained, with a yield of 48%.

### Comparative Example 2

### Preparation of (R)-4-ethoxy-4-oxobutan-2-yl-4-(4-cyano-2-methoxyphenyl)-2,8-dimethyl-5-oxo-1,4,5,6-tetrahydro-1,6-naphthyridine-3-carboxylate (structure as follows):

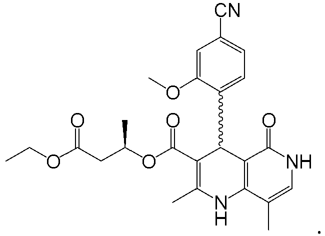

Following the experimental method of Example 2, except that a chiral catalyst was replaced with an equal amount of glacial acetic acid. A titled compound (S:R ratio = 58:42) was obtained, with a yield of 90.0%.

### Comparative Example 3

### Synthesis of 4-(4-cyano-2-methoxyphenyl)-2,8-dimethyl-5-oxo-1,4,5,6-tetrahydro-1,6-naphthyridine-3-carboxylic acid 2-cyanoethyl ester (structure as follows) by using a chiral catalyst:

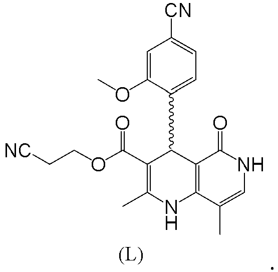

Following the experimental method of Example 2, except that 2-(4-cyano-2-methoxybenzylidene)-3-oxobutanoic acid 2-cyanoethyl ester was reacted with the compound of Formula 4 under catalysis by (S)-(+)-1,1'-binaphthyl-2,2'-diyl hydrogenphosphate. A titled compound (S:R ratio = 50:50) was obtained, with a yield of 87.0%.

Through the procedure of the above examples and comparative examples, the comparative results are summarized in Table 1.

**Table 1 Comparative results of examples and comparative examples**

| Example No. | Reactant | Target compound | Reaction conditions | S/R ratio of target compound | Yield |
|---|---|---|---|---|---|
| Example 2 | 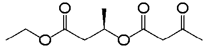 | 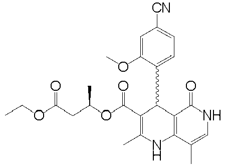 | Chiral catalyst-catalyzed reaction | 85:15 | 94.1 % |
| Example 7 | 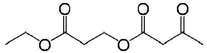 | 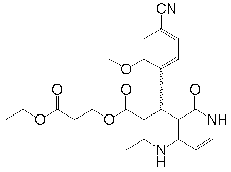 | Chiral catalyst-catalyzed reaction | 75:25 | 92.1 % |
| Example 8 | 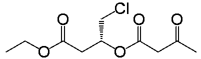 | 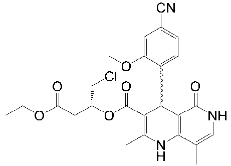 | Chiral catalyst-catalyzed reaction | 78:22 | 90.5 % |
| Example 9 | 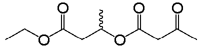 | 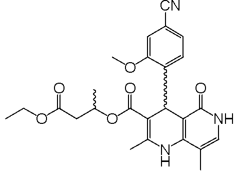 | Chiral catalyst-catalyzed reaction | 80:20 | 93.5 % |
| Comparativ e Example 1 | 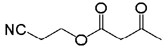 | 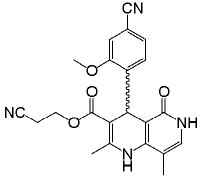 | High-pressure reaction | 50:50 | 88.0 % |
| Comparativ e Example 2 | 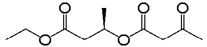 | 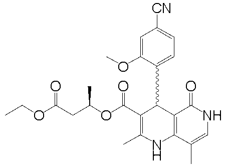 | Acetic acid-catalyzed reaction | 58:42 | 90.0 % |
| Comparativ e Example 3 | 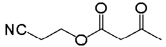 | 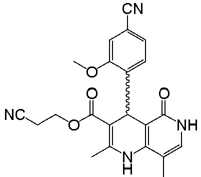 | Chiral catalyst-catalyzed reaction | 50:50 | 87.0 % |

From Table 1, it can be seen that in the examples of the present disclosure, the compound of Formula 2 having a unique diester structure is utilized in conjunction with a chiral catalyst to induce the formation of the chiral center in the target product. The method results in a target product with an S:R configuration ratio as high as 85:15, which is significantly superior to the comparative examples.

Although the present disclosure is described in detail in conjunction with the foregoing embodiments, they are only a part of, not all of, the embodiments of the present disclosure. Other embodiments can be obtained based on these embodiments without inventive efforts, and all of these embodiments shall fall within the scope of the present disclosure.

## Claims

1. A method for preparing finerenone, comprising the following steps:
I) mixing a compound of Formula 1, a compound of Formula 2, an organic base, acetic acid, and a first organic solvent, and conducting condensation, to obtain a compound of Formula 3;
II) mixing the compound of Formula 3, a compound of Formula 4, a chiral catalyst, and a second organic solvent, and conducting cyclization, to obtain a compound of Formula 5;
III) mixing the compound of Formula 5, triethyl orthoformate, concentrated sulfuric acid, and a third organic solvent, and conducting etherification, to obtain a compound of Formula 6;
IV) mixing the compound of Formula 6, a tartaric acid derivative, and a fourth organic solvent, conducting a first heat preservation by maintaining an obtained mixture at a first temperature, and then cooling a resulting heated product to a second temperature and conducting a second heat preservation, to obtain a salt of the tartaric acid derivative; and mixing the salt of the tartaric acid derivative, water, and a fifth organic solvent, and then regulating a pH value of a resulting mixture to 7.5 to 9.5 by using a basic pH regulator, to obtain a compound of Formula 7;
V) mixing the compound of Formula 7, a sixth organic solvent, and a basic compound, and conducting hydrolysis, to obtain a compound of Formula 8; and
VI) mixing the compound of Formula 8, N,N-carbonyldiimidazole (CDI), a seventh organic solvent, 4-dimethylaminopyridine (DMAP), and aqueous ammonia, and conducting amidation, to obtain the finerenone; 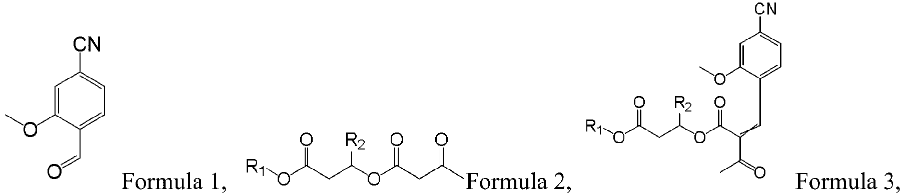 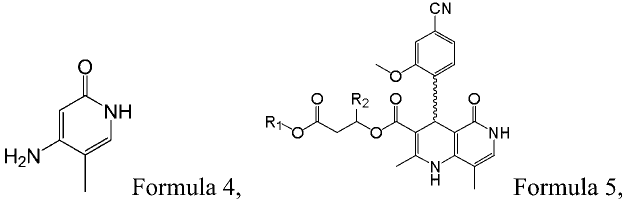 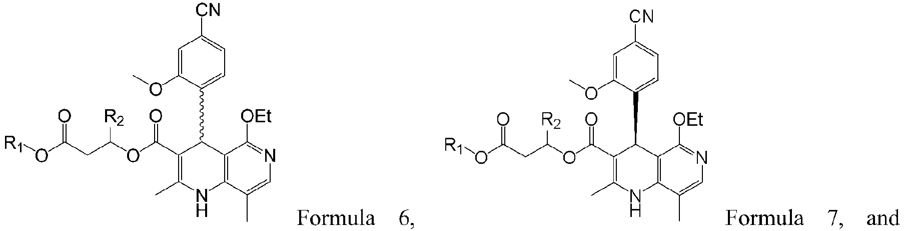 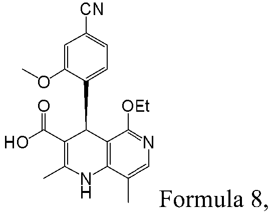 wherein in Formula 2, Formula 3, Formula 5, Formula 6, and Formula 7, R₁ is C1-10 alkyl, R₂ is selected from the group consisting of hydrogen, C1-10 alkyl, halogen-substituted C1-10 alkyl, and cyano-substituted C1-10 alkyl.

2. The method of claim 1, wherein in step I),
the organic base is at least one selected from the group consisting of piperidine, morpholine, and pyrrolidine;
the first organic solvent is at least one selected from the group consisting of methanol, ethanol, n-propanol, isopropanol, n-butanol, and sec-butanol;
a mass ratio of the compound of Formula 1, the organic base, the acetic acid, and the compound of Formula 2 is in a range of 1 : 0.03-0.2 : 0.02-0.2 : 1.0-2.0; and
the condensation is conducted at a temperature of 20°C to 50°C.

3. The method of claim 1 or 2, wherein a ratio of a volume of the first organic solvent to a mass of the compound of Formula 1 is in a range of 5 mL:1 g to 20 mL:1 g.

4. The method of claim 1, wherein in step II),
the chiral catalyst is selected from the group consisting of D-(+)-10-camphorsulfonic acid and (S)-(+)-1,1'-binaphthyl-2,2'-diyl hydrogenphosphate;
the second organic solvent is at least one selected from the group consisting of isopropanol, tert-butanol, sec-butanol, and sec-amyl alcohol;
a mass ratio of the compound of Formula 3, the compound of Formula 4, and the chiral catalyst is in a range of 1 : 0.2-1.2 : 0.2-2.0; and
the cyclization is conducted at a temperature of 70°C to 120°C.

5. The method of claim 1 or 4, wherein a ratio of a volume of the second organic solvent to a mass of the compound of Formula 3 is in a range of 5 mL:1 g to 20 mL:1 g.

6. The method of claim 1, wherein in step III),
the third organic solvent is at least one selected from the group consisting of *N,N-*dimethylformamide (DMF), *N*,*N*-dimethylacetamide (DMA), and *N*-methylpyrrolidone (NMP);
a mass ratio of the triethyl orthoformate to the compound of Formula 5 is in a range of 1:1 to 5:1;
the concentrated sulfuric acid has a mass percentage content of 98%, and a mass ratio of the compound of Formula 5 to the concentrated sulfuric acid is in a range of 1:0.02 to 1:0.3; and
the etherification is conducted at a temperature of 80°C to 130°C.

7. The method of claim 1 or 6, wherein a ratio of a volume of the third organic solvent to a mass of the compound of Formula 5 is in a range of 2 mL:1 g to 10 mL:1 g.

8. The method of claim 1, wherein in step IV), the tartaric acid derivative is selected from the group consisting of dibenzoyl-L-tartaric acid, L-di(*p*-toluoyl)tartaric acid, dibenzoyl-D-tartaric acid, and D-di(*p*-toluoyl)tartaric acid; a mass ratio of the compound of Formula 6 to the tartaric acid derivative is in a range of 1:0.4 to 1:1.2; the fourth organic solvent is at least one selected from the group consisting of methanol, ethanol, isopropanol, ethyl acetate, n-propyl acetate, isopropyl acetate, butyl acetate, acetone, and butanone; the first temperature is in a range of 30°C to 80°C, and the first heat preservation is conducted for 1 hour to 5 hours; the second temperature is in a range of 0°C to 25°C, and the second heat preservation is conducted for 1 hour to 24 hours; and
the fifth organic solvent is at least one or more selected from the group consisting of ethanol, methanol, isopropanol, n-butanol, and sec-butanol; a volume ratio of the fifth organic solvent to the water is in a range of 1:3 to 1:10; and the basic pH regulator is at least one selected from the group consisting of potassium carbonate, sodium carbonate, sodium bicarbonate, sodium phosphate, and potassium phosphate.

9. The method of claim 1 or 8, wherein a ratio of a volume of the fourth organic solvent to a mass of the compound of Formula 6 is in a range of 10 mL:1 g to 30 mL:1 g; and
a ratio of a volume of the fifth organic solvent to a mass of the compound of Formula 6 is in a range of 10 mL:1 g to 30 mL:1 g.

10. The method of claim 1, wherein in step V),
the basic compound comprises at least one selected from the group consisting of sodium hydroxide and potassium hydroxide, and a mass ratio of the basic compound to the compound of Formula 7 is in a range of 0.2:1 to 2.0:1;
the sixth organic solvent is at least one selected from the group consisting of methanol, ethanol, dichloromethane (DCM), and ethylene glycol dimethyl ether (DME); and
the hydrolysis is conducted at a temperature of 0°C to 30°C.

11. The method of claim 1 or 10, wherein a ratio of a volume of the sixth organic solvent to a mass of the compound of Formula 7 is in a range of 5 mL:1 g to 30 mL:1 g.

12. The method of claim 1, wherein in step VI),
the seventh organic solvent is at least one selected from the group consisting of DMF, DMA, NMP, and tetrahydrofuran (THF);
the aqueous ammonia has a mass percentage content of 25% to 28%;
a mass ratio of the compound of Formula 8, the CDI, the DMAP, and the aqueous ammonia is in a range of 1 : 0.3-1.5 : 0.02-0.2 : 3-10; and
the amidation is conducted at a temperature of 70°C to 120°C.

13. The method of claim 1 or 12, wherein a ratio of a volume of the seventh organic solvent to a mass of the compound of Formula 8 is in a range of 3 mL:1 g to 10 mL:1 g.

14. The method of claim 1, 8, or 10, wherein the method further comprises:
in step VI), after conducting the amidation to obtain an amidation reaction mixture, precipitating the amidation reaction mixture with water to obtain a crude product; and subjecting the crude product to purification with an alcoholic solvent to obtain pure finerenone, wherein the alcoholic solvent is at least one selected from the group consisting of methanol, an aqueous ethanol solution, and isopropanol, and ethanol in the aqueous ethanol solution has a mass percentage content of 95% to 100%; and
in step V), after conducting the hydrolysis to obtain a hydrolysis reaction mixture, neutralizing the hydrolysis reaction mixture with an acid, and precipitating a resulting neutralized product with water to obtain the compound of Formula 8, wherein the acid is at least one selected from the group consisting of hydrochloric acid, sulfuric acid, acetic acid, and formic acid.

15. The method of claim 14, wherein the purification comprises: dissolving the crude product in the alcoholic solvent with heating to obtain a crude product solution, and subjecting the crude product solution to concentration, cooling crystallization, solid-liquid separation, and drying in sequence, to obtain the pure finerenone, wherein a volume ratio of a concentrated solution obtained after the concentration to the crude product solution is in a range of 1:5 to 1:7, and the cooling crystallization is conducted at an initial temperature of 50°C to 60°C, an endpoint temperature of -5°C to 5°C, and a cooling rate of 1°C/min to 3°C/min.

16. Finerenone prepared by the method of any one of claims 1 to 15, wherein the finerenone exhibits an enantiomeric excess (ee) value greater than 99.9%.

17. A finerenone intermediate prepared by the method of any one of claims 1 to 11, wherein the finerenone intermediate has a structure selected from the group consisting of the Formula 3, the Formula 5 the Formula 6 and the Formula 7: 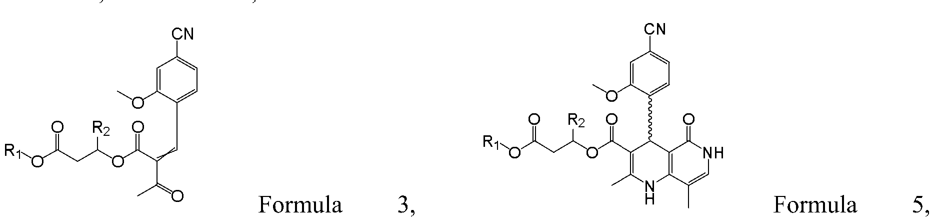 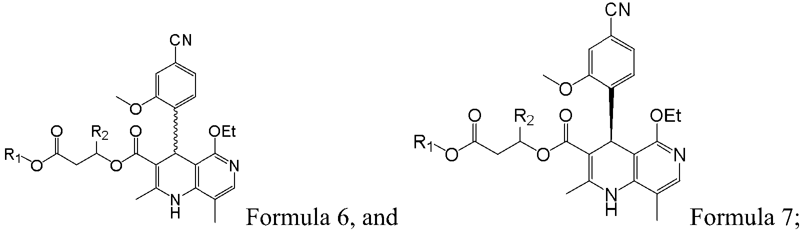 wherein in the Formula 3, the Formula 5, the Formula 6, and the Formula 7, R₁ is C1-10 alkyl, R₂ is selected from the group consisting of hydrogen, C1-10 alkyl, halogen-substituted C1-10 alkyl, and cyano-substituted C1-10 alkyl.
